# EUROPÄISCHE PATENTSCHRIFT

(11) **EP 3 643 224 B1**
(45) Veröffentlichungstag und Bekanntmachung des Hinweises auf die Patenterteilung: **05.05.2021**
(21) Anmeldenummer: 19205066.4
(22) Anmeldetag: 24.10.2019
(51) Int. Cl.: A61B 5/01, A61F 5/443, A61F 5/448

(54) **VORRICHTUNG ZUR BEFESTIGUNG EINER BASISPLATTE VON STOMAVORRICHTUNGEN SOWIE EIN VERFAHREN ZU DEREN ANWENDUNG**
DEVICE FOR FIXING A BASE PLATE OF STOMA DEVICES AND METHOD FOR THE APPLICATION THEREOF
DISPOSITIF DE FIXATION D'UNE PLAQUE DE BASE DE DISPOSITIFS STOMA ET SON PROCÉDÉ D'APPLICATION

(30) Priorität: 24.10.2018 DE 102018126465
(43) Veröffentlichungstag der Anmeldung: 29.04.2020
(73) Patentinhaber: Riedel, Klaus, 02708 Dürrhennersdorf (DE)
(72) Erfinder: Riedel, Klaus, 02708 Dürrhennersdorf (DE)
(74) Vertreter: Hofmann, Klaus

(56) Entgegenhaltungen:
- EP-A1- 1 815 828
- WO-A2-2005/070360
- GB-A- 2 290 974
- JP-U- S5 398 664
- US-A- 4 784 656

## Beschreibung

Die Erfindung betrifft eine Vorrichtung zur Befestigung einer Basisplatte auf der Haut von betroffenen Personen bei Stomavorrichtungen, die aus einem Auffangbeutel und einer selbsthaftenden Basisplatte bestehen sowie ein Verfahren zu deren Anwendung.

Bei der medizinischen Versorgung von betroffenen Personen mit einem Stoma nach einer Darmoperation ist es erforderlich, dass die Betroffenen eine sichere Stomaversorgung erhalten, bei der sie so wenig wie möglich Komplikationen ausgesetzt werden.

Eine Stomaversorgung besteht immer aus einer auf der Bauchdecke aufzuklebenden Basisplatte und einem daran befestigten Auffangbeutel, der der Aufnahme der Ausscheidungen dient. Man unterscheidet ein- und zweiteilige Systeme.

Bei den einteiligen Systemen sind Basisplatte und Auffangbeutel fest miteinander verbunden und können nur gemeinsam gewechselt werden.

Zweiteilige Systeme sind dadurch gekennzeichnet, dass Basisplatte und Auffangbeutel getrennte Einheiten darstellen. Das bedeutet, dass die Platte auf die Haut geklebt wird und der Beutel nachträglich mittels eines Kopplungsringes oder einer Klebefläche damit verbunden wird.

Bei zweiteiligen Systemen mit einer Rastverbindung sind zur Verbindung von Basisplatte und Beutel an beiden Teilen jeweils Kopplungsringe aus Kunststoff vorhanden, die ineinander rastbar sind. Der rastbare Kopplungsring steht dabei an der Basisplatte mehrere Millimeter über der Fläche der Basisplatte vor und bildet einen Überstand.

Bei einteiligen Systemen mit Adhäsivkopplung ist dieser Überstand meist nur gering oder gar nicht vorhanden.

Das zweiteilige Versorgungssystem ermöglicht einen Verbleib der Platte auf dem Bauch eines Betroffenen, wenn der Beutel aus Hygienegründen gewechselt werden muss.

Eine zweiteilige Stomaversorgung wird beispielsweise in DE 378 0269 T2 beschrieben. Darin wird eine verbesserte Verbindung zwischen der Basisplatte und dem abnehmbaren Auffangbeutel angestrebt. Ersichtlich ist dabei, dass die eingesetzte Schraubverbindung aus der hier rechteckigen Basisplatte hervorsteht.

Das erkannte Problem einer sicheren Haftung an für den Betroffenen empfindlichen Stellen wird beispielsweise in GB 1 021 145 A angesprochen. Die hier beschriebene Lösung betrifft aber nicht die Befestigung der Basisplatte auf der Haut. Zusätzliche an der Stomavorrichtung befestigte Gürtel sollen einen sicheren Halt unterstützen.

Die US 4 710 182 A beschreibt eine Ostomievorrichtung, die eine Basisplatte für eine klebende Befestigung an einem Betroffenen aufweist. Sie enthält einen Hautbarrierering, einen mikroporösen Flecken und einen Verbindungsring, der den Hautbarrierering, den mikroporösen Flecken und den Beutel miteinander verbindet. Der Hautbarrierering wird aus einem weichen, biegbaren, wasserabsorbierendem Material gefertigt.

Die GB 2 290 974 A beschreibt ein körperseitiges Ostomieteil, welches Teil einer zweiteiligen Stomavorrichtung sein kann, also einer Basisplatte sowie eines verbindbaren Aufnahmebeutels. Gemäß einem Aspekt der Erfindung wird ein körperseitiges Ostomieteil bereitgestellt, bei dem eine umlaufende Rippe eine formbare Masse eines nicht hypoallergenen, im Wesentlichen nicht speicherhaften, kittartigen Klebstoffs umgibt und einschließt. Diese ist so beschaffen, dass sie mit den Fingern verformbar ist, so dass sie eine donutartige Polster- und Schutzmasse bildet, die das Stoma umgibt.

In WO 2005/070360 A2 werden Haftklebstoffzusammensetzungen beschrieben, die für verschiedene medizinische Anwendungen geeignet sind und insbesondere zur Verwendung für die Haftung auf der Haut geeignet sind, insbesondere auf dem Gebiet der Wund- oder der Stomaversorgung. Die verwendeten Klebstoffe besitzen eine gummiartige, elastomere Basis, welche bei Raumtemperaturen nicht fließfähig sind.

JP S53 98664 U betrifft einen Topf, der mit einem elektrischen Heizelement ausgestattet ist, ein Plug-in-Thermometer, das elektrisch mit dem elektrischen Heizelement des Topfes verbunden und von diesem elektrisch abnehmbar ist. Der Topf ist mit einem Thermometer ausgestattet, das die Temperatur des Topfes erfasst und einstellt. Ein elektrischer Herd besitzt eine Hitzeschildplatte, die sich an der Unterseite des Topfes befindet und einen Spalt zwischen dem Topf und der Hitzeschildplatte aufweist und vier Teile auf der Hitzeschildplatte bildet, in denen ein Einsteckmodul aufbewahrt werden kann.

Meist besitzen die zweiteiligen Basisplatten eine Kopplungsvorrichtung für den abnehmbaren Beutel, die auf einer Hautschutzgrundlage befestigt ist. Die Hautschutzgrundlage, welche meist aus Kunststoffmaterial besteht, ist um den direkten Stomabereich mit einer selbsthaftenden Spezialkleberschicht versehen. Aus diesem Grunde ist dieser Klebebereich auch meist rund ausgebildet. Der Spezialkleber wird vorzugsweise so gewählt, dass er bei Körpertemperatur die besten Klebeeigenschaften aufweist.

Außerhalb des runden, direkten Stoma-Spezialkleberbereiches kann die Hautschutzgrundlage zusätzlich mit einer selbsthaftenden Beschichtung versehen sein, die eine geringere Klebefähigkeit besitz. Diese ist oft rechteckig, meist mit einem Schutzpapier versehen und dient einer zusätzlichen Sicherheit.

Aufgrund des bekannten Problemes einer ungenügenden Klebefähigkeit werden große Anstrengungen bei der Erforschung von selbsthaftenden Klebstofflösungen unternommen.

In allen beschriebenen Lösungen wird davon ausgegangen, dass eine selbsthaftende Basisplatte mit einem geeigneten Kleber einen sicheren und dichten Halt gewährleistet. Vorgeschlagen wird auch, dass zusätzliche Gürtel zu tragen sind.

Beim Andrücken der Basisplatte von Hand ist es nicht möglich, die gesamte Fläche etwa für zwei Minuten vollflächig und gleichmäßig mit der Hand anzudrücken. Eine nicht vollflächige Verbindung führt in kürzester Zeit zum Lösen der Platte, was immer mit einem Totalverlust des ganzen Systems einhergeht.

Der derzeitige Standard ist relativ einfach, aber mit erheblichen Risiken verbunden. In der einschlägigen Literatur oder in Internetforen werden oft einfache Lösungen beschrieben, die jedoch sehr viele Probleme enthalten.

So wird beispielsweise empfohlen, die Basisplatte vor Verwendung geraume Zeit unter der Achselhöhle auf Temperatur zu bringen. Diese Anwendung verfehlt ihre Wirkung schon dadurch, dass in der Zeit des Anlegens der Basisplatte diese die Körpertemperatur nicht halten kann.

Der ganze Vorgang hat auch einen nicht zu unterschätzenden ökonomischen Hintergrund. Die Kosten für eine Basisplatte liegen derzeit bei etwa 12,- bis 17,- € je Stück. Bedenkt man, dass eine fehlerhaft angebrachte, also nicht verwendbare Basisplatte unter Umständen täglich mehrfach erneuert werden muss, kann der materielle Schaden sehr schnell erfasst werden. Die seelische und körperliche Belastung von betroffenen Personen, welche sich sehr viel in öffentlichen Räumen bewegen, ist zusätzlich sehr hoch einzuschätzen.

Aufgabe der Erfindung ist es, eine Vorrichtung, ein Verfahren zu deren Anwendung zur sicheren und schonenden Befestigung einer Basisplatte auf der Haut von Betroffenen bei Stomavorrichtungen bereitzustellen, die aus einem Beutel und einer selbsthaftenden Basisplatte bestehen. Die Vorrichtung soll einfach aufgebaut, kostengünstig herstellbar und für jeden Benutzer einfach anwendbar sein. Insbesondere soll eine gleichmäßige Druckverteilung in einem optimalen Temperaturbereich ermöglicht werden.

Erfindungsgemäß wird die Aufgabe durch die Merkmale der Ansprüche 1, 11 gelöst. Ausgestaltende Merkmale sind in den Unteransprüchen 2 bis 10 beschrieben.

Die Vorrichtung dient der Befestigung einer Basisplatte bei Stomavorrichtungen, am effektivsten mit zweiteiligen Kopplungssystemen, die aus einem festen oder abnehmbaren Beutel und einer selbsthaftenden Basisplatte bestehen. Die Verbindung bei zweiteiligen Kopplungssysteme kann mittels zweier rastbaren Kopplungsringe oder mittels einer Klebeverbindung erfolgen. Im Falle einer Rastverbindung ragt der Kopplungsring an der Basisplatte etwas über die Fläche der Basisplatte und bildet einen Überstand.

Erfindungsgemäß besteht die Vorrichtung aus einer Wärmespeicherplatte, die an der bei der Verwendung der Vorrichtung der Basisplatte abgewandten Seite eine Aufnahmeöffnung für ein Thermometer besitzt. In dieser Aufnahmeöffnung sind Mittel zur Fixierung des Thermometers vorgesehen. Dabei ist darauf zu achten, dass das Thermometer so in Kontakt mit der Aufnahmeöffnung ist, dass ein ungehinderter Temperaturaustausch erfolgen kann. Insbesondere sollte gewährleistet werden, dass der Kontakt zumindest an der Rückseite erfolgt, um die gesamte Grundfläche des Thermometers für eine korrekte und schnell wirkende Temperaturanzeige auszunutzen.

Die Wärmespeicherplatte ist in ihren Eigenschaften hinsichtlich Material, Dimensionierung und Wärmeleitfähigkeit so zu wählen, dass beim Auflegen der Vorrichtung auf die Rückseite der mit Klebstoff behafteten Basisplatte die Temperaturabsenkung an der Oberfläche des Wärmespeicherplatte innerhalb von 2 min nicht mehr als 5°C beträgt. Die Auflage der erfindungsgemäßen Vorrichtung auf die Basisplatte soll solange erfolgen, bis sich die Fläche der mit Spezialklebstoff belegten Basisplatte auf die Körpertemperatur erwärmt hat. Es hat sich herausgestellt, dass dies in mindestens 2 min erfolgt.

Dabei sollte die Wärmespeicherplatte bei einer Temperaturabsenkung von 5°C eine Wärmemenge von mindestens 0,3 Wh abgeben und in mehr als 2 min einen Wärmestrom von mindestens 2 W abgeben können.

Die Wärmespeicherplatte sollte wegen des guten Wärmeüberganges aus Metall bestehen. Aus Fertigungs- und Anwendungsgründen ist eine Ausführung aus Aluminium bzw. einer Aluminiumlegierung vorteilhaft. Ebenfalls möglich sind Wärmespeicherplatten aus Edelstählen. Je kleiner der Durchmesser der Wärmespeicherplatte ist, umso dicker müsste sie demnach sein.

Um eine ausreichende Wärmespeicherung zu gewährleisten, sollte eine metallische Wärmespeicherplatte ein Volumen von mindestens 50 cm³, optimal von mehr als 90 cm³ besitzen.

Da es sich bei der erfindungsgemäßen Vorrichtung um ein medizienisches Gerät handelt, sind die geltenden gesetzlichen Bestimmungen zu beachten. Aus diesem Grunde sollten alle verwendeten Teile aus bleifreien Materialien bestehen, wasserdicht sowie harz- und säurefest sein. Außerdem sind die eingesetzten Materialien hinsichtlich ihrer Möglichkeiten zur Wärmespeicherung und Wärmeabgabe beim Einsatz optimal zu gestalten. Diese Bedingungen werden von nichtrostenden Metallen erfüllt. Da Edelstahl für die Handhabung relativ schwer ist, hat sich eine Aluminiumlegierung, beispielsweise AlMg3, als vorteilhaft erwiesen.

Besonders wirksam ist die Vorrichtung, wenn die Grundfläche der Wärmespeicherplatte mindestens der dem Körper zugewandten Fläche einer Spezialkleberschicht der Basisplatte entspricht. Am besten ist es, wenn sie identisch sind.

Die Wärmespeicherplatte wird aufgrund der meist runden Form der Spezialkleberschicht der üblichen Basisplatten auch rund ausgebildet werden. Sie kann aber auch oval oder rechteckig sein. Die Aufnahmeöffnung für das Thermometer wird aufgrund meist runder Thermometer auch rund ausgebildet sein. Schon aus Fertigungsgründen ist das vorteilhaft. Andere Formen sind jedoch ebenfalls möglich.

An der Basisplatte zugewandten Andruckseite ist eine Profilaussparung vorhanden, die im Wesentlichen der äußeren Kontur eines an der Basisplatte vorhandenen Überstandes und deren Tiefe minimal der Höhe dieses Überstandes entspricht. Dies ist insbesondere bei einer Rastverbindung von Basisplatte und Beutel erforderlich, bei der ein an der Basisplatte vorhandener Kopplungsring mehrere Millimeter übersteht.

Üblicherweise besitzen die Kopplungsringe bei Stomavorrichtungen mit 2-teiligen Kopplungssystemen einen Durchmesser zwischen 40 mm und 55 mm, maximal aber 70 mm.

Als Mittel zur Fixierung des Thermometers in der Aufnahmeöffnung kann ein Sicherungsring dienen, der in eine im Umfang der Aufnahmeöffnung vorgesehene Ringnut einpassbar ist und das Thermometer fest auf die Rückseite der Aufnahmeöffnung presst.

Für eine leichte Entwässerung kann in der Wärmespeicherplatte eine Entwässerungsbohrung enthalten sein, die in die Aufnahmeöffnung mündet und dort eine einfache Ableitung bzw. ein leichtes Ausschütteln des bei der Erwärmung der Wärmespeicherplatte enthaltenen Wasser ermöglicht.

Eine weitere Möglichkeit besteht darin, dass zur Fixierung des Thermometers in der Aufnahmeöffnung ein wärmeleitender Kleber eingesetzt wird. Die wärmeleitenden Klebstoffe sind Kunstharze, die mit entsprechenden metallischen oder anorganischen Füllstoffen angereichert sind. Im Vergleich zu Wärmeleitpasten haben wärmeleitende Klebstoffe den Vorteil, dass sie nicht nur die hohe Wärmeenergie abführen, sondern gleichzeitig zur Fixierung und Befestigung von Bauteilen dienen.

Das Verfahren zur Anwendung einer erfindungsgemäßen Vorrichtung besteht darin, dass die Vorrichtung möglichst flächendeckend mit einer auf etwa 40°C erwärmten Wärmespeicherplatte mindestens 2 min auf die Rückseite einer mit Spezialklebstoff belegten Basisplatte einer Stomavorrichtung gelegt wird, bis die Temperatur der Spezialkleberschicht die Körpertemperatur erreicht hat. Nach Abkühlung der Wärmespeicherplatte um mindestens 5°C wird die Vorrichtung wieder entfernt. Optimal ist eine Abkühlung um etwa 10°C. Dabei hat sich der Spezialklebstoff auf die Körpertemperatur erhöht, wo er seine beste Klebewirkung entfaltet. Im weiteren Verlauf kühlt sich der Spezialklebstoff ab und härtet wieder aus.

Eine separate Vorwärmung der Basisplatte mittels der erfindungsgemäßen Vorrichtung kann wahlweise erfolgen und wirkt sich positiv auf das Klebeergebnis aus.

Erstmals wird eine Vorrichtung mit einer Wärmespeicherplatte zur sicheren Befestigung einer Basisplatte von Stomavorrichtungen mit einem abnehmbaren Beutel und einer selbsthaftenden Basisplatte bestehen, benutzt. Die Wärmespeicherplatte besitzt dabei an der bei der Verwendung der Vorrichtung der Basisplatte abgewandten Seite eine Aufnahmeöffnung für ein Thermometer und Mittel zur Fixierung des Thermometers in der Aufnahmeöffnung.

Die erfindungsgemäße Vorrichtung ist für Stomavorrichtungen mit zweiteiligen Kopplungssystemen, die aus einem abnehmbaren Beutel und einer separaten, selbsthaftenden Basisplatte bestehen, einsetzbar. Bei einteiligen Stomavorrichtungen ist der feste Beutel für eine gleichmäßige Wärmeübertragung auf die Klebefläche hinderlich. Da dieser Beutel aber nur aus einer sehr dünnen Folie besteht, kann die Wärmeübertragung auch durch diese Folie erfolgen. Das Klebeergebnis wird dabei nicht so optimal sein, wie bei 2-teiligen Kopplungssystemen, es wird aber immer noch ausreichend sein. Die Wärmespeicherplatte arbeitet vorzugsweise in einem Temperaturbereich zwischen 40°C und 35°C, maximal bis 30°C. Sie beeinflusst die Klebstoffeigenschaften der auf der Haut haftenden Basisplatte derart positiv, so dass sich deren Adhäsivität erhöht.

Auf Basisplatten werden druckempfindliche Spezialkleberzusammensetzungen verwendet. Diese Kleberzusammensetzungen enthalten in einer kautschukartigen Elastomerbasis dispergierte, wasserlösliche und in Wasser quellbare Hydrokolloide.

Die dispergierten löslichen Thermoplaste dieses physikalisch abbindenden Schmelzklebstoffes lassen sich durch Erwärmung verflüssigen und erstarren wieder durch Erkalten. Das Abkühlen des Schmelzklebstoffes hat zur Folge, dass sich die Polymerketten einander annähern und so die Beweglichkeit der Polymerketten eingeschränkt wird.

Zwischen den Polymerketten bilden sich physikalische Wechselwirkungen aus, die den Grad der Kohäsion des Klebstoffes im verfestigten Zustand bestimmen. Auf diese Weise kann die betroffene Person den Andruckvorgang in einem für den Spezialklebstoff optimalen Temperaturbereich durchführen.

Ein längeres Abkühlen um bis zu 10°C wird die Klebewirkung noch verstärken, da die eingesetzten Kleber noch besser aushärten können, jedoch wird sich die erforderliche Zeit dadurch auf bis zu 10 min verlängern.

Eine Ausgangstemperatur von über 40°C sollte möglichst vermieden werden. Der Klebeprozess würde bei den derzeitig verwendeten Klebern dadurch negativ beeinflusst, da die Klebefähigkeit des eingesetzten Schmelzklebstoffes bei viel mehr als 40°C nicht optimal funktioniert. Dabei ist zu gewährleisten, dass die Temperatur an der Oberfläche der Spezialkleberschicht etwa die Körpertemperatur erreicht. Bei der Verwendung anderer Kleber kann der optimale Temperaturbereich entsprechend angepasst werden.

Die Erfindung sichert das Anbringen einer Basisplatte mit vorzugsweise zweiteiligen Kopplungsystemen am Körper, an die ein Auffangbeutel befestigt ist. Die Vorrichtung ist einfach aufgebaut, kostengünstig herstellbar und für jeden Benutzer einfach anwendbar. Insbesondere wird eine gleichmäßige Druckverteilung in einem für die eingesetzten Kleber optimalen Temperaturbereich ermöglicht.

Durch eine veränderlich herstellbare Form der Profilaussparung können die unterschiedlichen Maße verschiedener Hersteller berücksichtigt und dementsprechend angepasst werden.

Ökonomisch betrachtet bietet sich für den Betroffenen eine günstige Alternative. Die Kosten für eingesparte Basisplatten von derzeit etwa 12,- bis 17,- € je Stück sind bei der Anwendung der erfindungsgemäßen Vorrichtung bei unbegrenzter Verwendungszeit schon nach kurzer Zeit amortisiert.
Diese Vorrichtung wird den betroffenen Personen, dem Personal in den Kliniken, den nicht ausgebildeten Familienangehörigen und den Pflegekräften ihre Tätigkeit erleichtern. Mit jeder Basisplatte, welche sich durch lange Haltbarkeit am Körper auszeichnet, werden Betroffene und auch Krankenkassen finanziell entlastet.

Nachfolgend wird die Erfindung an einem Ausführungsbeispiel näher erläutert. Es zeigen:
- Fig. 1: Explosionsansicht einer Vorrichtung
- Fig. 2: Draufsicht und Schnitt A-A durch eine Vorrichtung mit Detail B und C
- Fig. 3: Vorrichtung mit einer aufsetzbaren Basisplatte
- Fig. 4: Vorrichtung mit aufgesetzter Basisplatte

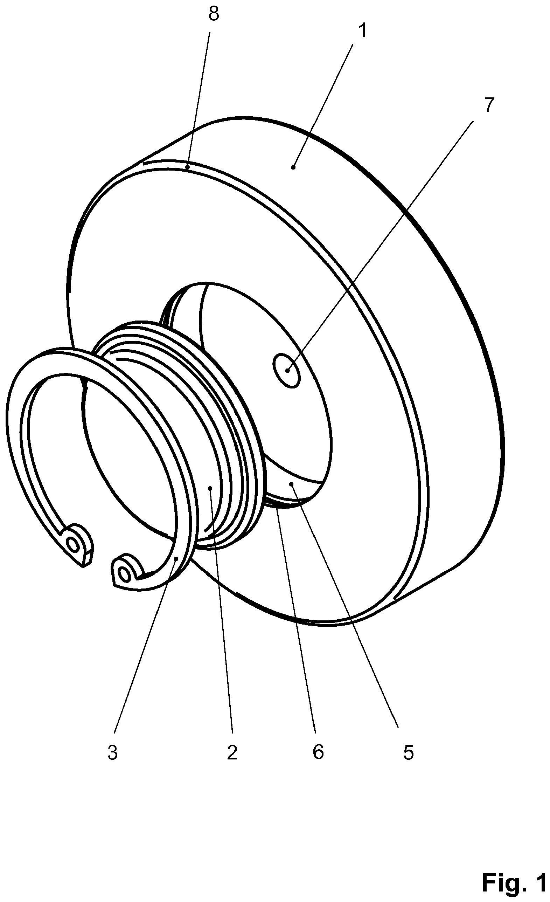

Bei dem nach den Fig. 1 bis 4 beschriebenen Ausführungsbeispiel handelt es sich um eine mögliche Variante einer Vorrichtung für eine Basisplatte 10 bei zweiteiligen Stomavorrichtungen, bei denen auf einer Hautschutzunterlage 14 ein Kopplungring 12 aus Kunststoff befestigt ist. Der Kopplungsring 12 hat einen Durchmesser von etwa 50 mm, ist einige mm breit und steht etwa 2,5 mm über der Hautschutzunterlage 14 vor. Auf der Hautschutzunterlage 14 ist eine etwa 30 mm breite Spezialkleberschicht 13 aufgebracht, der um das Stoma auf die Haut zu kleben ist.

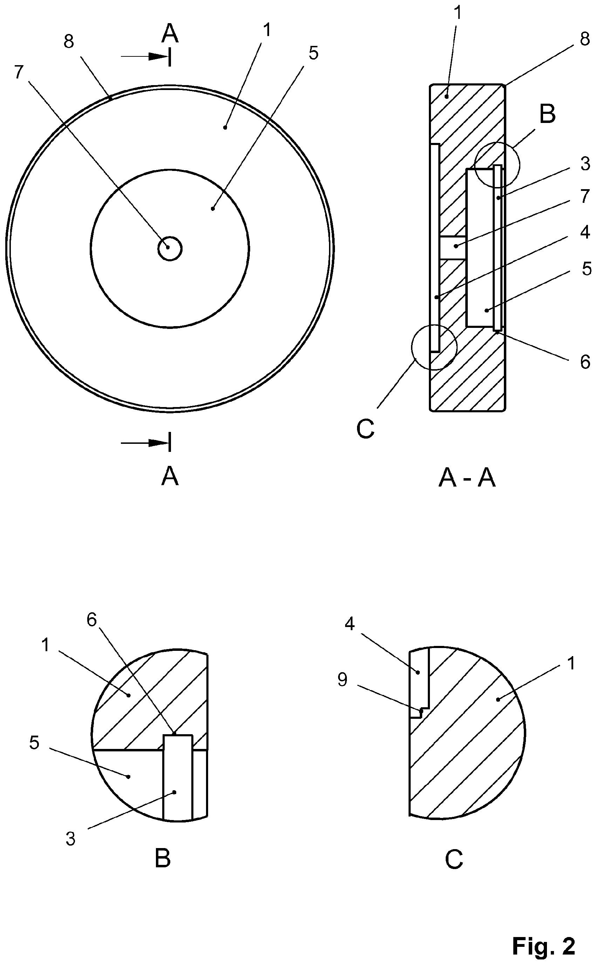

Die Fig. 1 und Fig. 2 zeigen eine erfindungsgemäße Vorrichtung, bei der eine Wärmespeicherplatte 1 an der Basisplatte 10 abgewandten Seite eine Aufnahmeöffnung 5 für ein Thermometer 2 besitzt. Die Wärmespeicherplatte 1 ist eine runde Platte aus einer Aluminiumlegierung, hier AlMg3, mit einem Durchmesser von 79 mm, einer Dicke von 19,5 mm und einer umlaufenden Abkantung 8. Diese Maße sind je nach Ausführung der eingesetzten Basisplatte 10 variabel anpassbar. Das Volumen der Wärmespeicherplatte 1 beträgt etwa 100 cm³.

Die eingesetzte Aluminiumlegierung ist metallisch gut leidend, korrosionsfrei und einfach bearbeitbar.

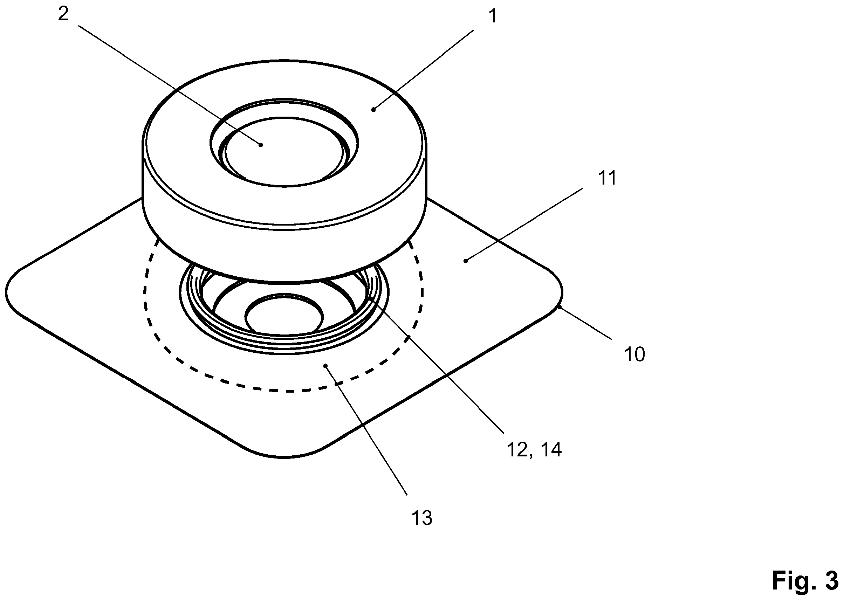

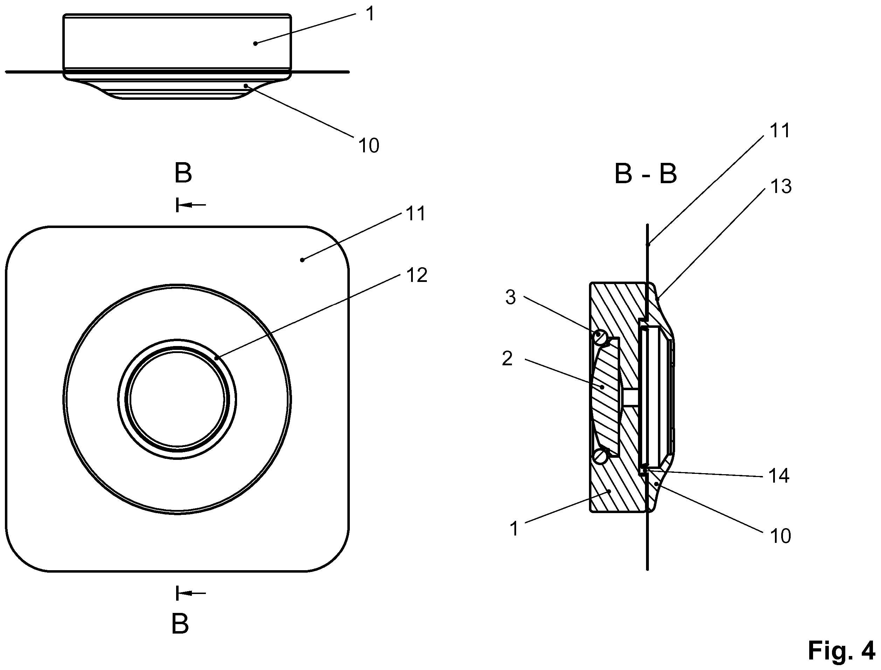

Die Fig. 3 zeigt eine Vorrichtung, wie sie auf eine Basisplatte 10 aufsetzbar ist. In Fig. 4 ist eine auf die Basisplatte 10 aufgesetzte Vorrichtung in 2 Ansichten sowie im Schnitt B-B dargestellt.

Die Wärmespeicherplatte 1 funktioniert als Wärmespeicher. Da der eingesetzten Spezialkleber seine beste Klebewirkung bei Körpertemperatur besitzt, ist ein wasserdichtes Thermometer 2 eingebaut. Dadurch wird es möglich, die Temperatur genau im vorgeschriebenen Bereich zu halten.

Das Thermometer 2 wird mit einem Sicherungsring 3 fixiert, der innen im Umfang der Aufnahmeöffnung 5 in einer in Detail B ersichtlichen Ringnut 6 angeordnet ist. Die Abmessungen sind dabei so bemessen, dass das Thermometer 2 fest in der Aufnahmeöffnung 5 sitzt und ein ungehinderter Temperaturaustausch ermöglicht wird.

Der Kopplungsring 12 der Basisplatte 10 besitzt eine zylinderförmige Kontur. Bei manchen Kopplungsringen 12 ist diese Kontur auch seitlich stufig oder nach außen hin abgeflacht ausgebildet und bildet einen Überstand 14.

Auf der Gegenseite der Wärmespeicherplatte 1 ist deshalb eine auf den Überstand 14 des Kopplungsringes 12 der Basisplatte 10 abgestimmte Profilaussparung 4 eingearbeitet. Dementsprechend ist die Kontur der Aufnahmeöffnung 5 auch korrespondierend mit einer Profilierung 9 ausgebildet. Im Schnitt A-A ist eine zylinderförmige Kontur abgebildet. Das Detail C zeigt beispielsweise eine abgestufte Kontur. Die Profilaussparung 4 ist entsprechend der Höhe des Kopplungsringes 12 der Basisplatte 10 hier 2,5 mm tief.

Vorteilhaft ist es, wenn das eingearbeitete Profil in der Wärmespeicherplatte 1 und die äußere Kontur der Basisplatte 10 nahezu identisch sind. Das führt dazu, dass beim Drücken mit der Wärmespeicherplatte 1 auf die Basisplatte 10 gleichzeitig die gesamte Fläche der Spezialkleberschicht 13 auf den Körper gedrückt wird. Damit wird verhindert, dass sich an der Klebefläche nicht die kleinsten Falten bilden können.

In der Wärmespeicherplatte 1 ist eine durchgehende Entwässerungsbohrung 7 enthalten, die in die Aufnahmeöffnung 5 mündet und durch die enthaltenes Wasser leicht selbst entweichen oder ausgeschüttelt werden kann.

Außerhalb der runden, direkten Spezialkleberschicht 13 ist die Hautschutzunterlage 11 zusätzlich mit einer selbsthaftenden Beschichtung versehen, die eine geringere Klebefähigkeit besitz. Diese ist rechteckig ausgebildet und mit einem Schutzpapier versehen, ähnlich wie bei einem handelsüblichen Heftpflaster.

Die Vorgehensweise beim Anbringen einer Basisplatte 10 bei einem zweiteiligen Kopplungssystem mit Hilfe der erfindungsgemäßen Vorrichtung ist wie folgt:
Das Stomaversorgungssystem besteht aus einer separaten Basisplatte 10 als Hautschutz und einem Auffangbeutel. An beiden Teilen ist ein rastbarer Kopplungsring 12 befestigt, über den sie miteinander verbunden werden.

Nach der Reinigung und der Trocknung der peristomalen Haut um das Stoma wird der Hautschutz vorbereitet. Entweder der Hautschutz wird zugeschnitten oder bei modellierbarem Hautschutz entsprechend angepasst. Das Schutzpapier wird entfernt und die Basisplatte 10 wird nach Vorschrift des jeweiligen Herstellers aufgeklebt.

Um die eingesetzte Basisplatte 10 sicher und fest mit der Klebefläche der Spezialkleberschicht 13 um das Stoma aufzukleben, ist die Temperatur der Klebefläche äußerst wichtig. Bei kalter Haut oder bei kalten Umgebungstemperaturen dauert es sehr lange, bis sich die Spezialkleberschicht 13 erwärmt bzw. die Körpertemperatur annimmt. Das ist aber gerade die Voraussetzung dafür, dass der Spezialkleber seine beste Klebefähigkeit entfaltet.

Mit einem Warmwasserstrahl erwärmt man die Wärmespeicherplatte 1 auf ca. 40 Grad Celsius. Diese Temperatur wird auf dem Thermometer 2 angezeigt und kann von der betroffenen Person abgelesen werden. Nun wird die erwärmte Wärmespeicherplatte 1 mit der Seite der eingearbeiteten Profilaussparung 4 auf die am Körper befestigte Basisplatte 10 gedrückt. Nach etwa zwei Minuten hat die Klebefläche der Basisplatte durch die von der Wärmespeicherplatte 1 übertragene Wärme die Körpertemperatur und nach weiteren 2 Minuten etwa 34°C erreicht. Die Vorrichtung kann nun abgenommen werden. Ein festes Andrücken über die gesamte runde Fläche der Spezialkleberschicht 13 der Basisplatte 10 ermöglicht eine faltenfreie und dauerhafte Befestigung auf der Haut des Betroffenen.

Als günstig und ausreichend hat sich erwiesen, wenn die auf 40°C erwärmte Wärmespeicherplatte 1 bezüglich ihrer Eigenschaften zur Wärmespeicherung und Wärmeabgabe so gewählt wird, dass der Zeitraum, in dem sich die Wärmespeicherplatte 1 um 5°C abkühlt mehr als 2 min dauert. Die Wärmespeicherplatte 1 sollte aus Handhabungs- und Gewichtsgründen so klein wie möglich sei. Sie ist jedoch so groß zu wählen, dass ausreichend viel Wärmeenergie aufgenommen und ausreichend Wärme mindestens 2 min gehalten werden kann, bis sie unter die Körpertemperatur sinkt.

Die eingesetzte Wärmespeicherplatte 1 erfüllt bei der im vorliegenden Beispiel verwendeten Dimensionierung und dem hier eingesetzten Material die erforderlichen Bedingungen. Bei einem Volumen von etwa 100 cm³, einer Masse von ca. 270 g und einer spezifischen Wärmekapazität der Aluminiumlegierung von c = 0,89 kj (kg K)_¹ wird sie bei 5 Grad Abkühlung eine Wärmemenge von etwa 0,3 Wh abgeben. Dabei wird in der Einsatzzeit bei der Abkühlung durch die aufgelegte Hand sowie die Wärmestrahlung und Wärmeströmung durch die Umgebung ein Wärmestrom von mehr als 2 W übertragen.

Noch optimaler ist es, wenn eine Abkühlung um 10°C von 40°C auf etwa 30°C erfolgt, da die eingesetzten Spezialkleber noch besser aushärten können und die Klebeeigenschaften dadurch noch verbessert werden. Es liegt im Ermessen des Anwenders, einen für ihn hinsichtlich verfügbarer Zeit und Klebewirkung optimalen Bereich zu finden.

Der Anwender kann die Temperatur sowohl bei der Aufheizung der Wärmespeicherplatte 1 als auch beim Aufdrücken der Wärmespeicherplatte 1 auf die Basisplatte genau verfolgen. Eventuell sich in der Aufnahmeöffnung 5 befindliches Restwasser kann durch die Entwässerungsbohrung 7 entweichen bzw. leicht ausgeschüttelt werden. Ein wasserdichtes und korrosionsfreies Gehäuse des Thermometers 2 garantiert eine lange Lebensdauer.

Anschließend ist der Auffangbeutel nach den Hinweisen des Herstellers anzubringen.

Durch diese Vorgehensweise wird eine dauerhafte und größtmögliche Haftung der Verbindung zwischen Körper und Basisplatte 10 geschaffen.

### Bezugszeichenaufstellung

- 1: Wärmespeicherplatte
- 2: Thermometer
- 3: Sicherungsring
- 4: Profilaussparung
- 5: Aufnahmeöffnung
- 6: Ringnut
- 7: Entwässerungsbohrung
- 8: Abkantung
- 9: Profilierung
- 10: Basisplatte
- 11: Hautschutzunterlage
- 12: Kopplungsring
- 13: Spezialkleberschicht
- 14: Überstand

## Patentansprüche

1. Vorrichtung zur Befestigung einer Basisplatte von Stomavorrichtungen mit zweiteiligen Kopplungssystemen, die aus einem abnehmbaren Beutel und einer separaten selbsthaftenden Basisplatte bestehen, auf der Haut von betroffenen Personen, wobei die Vorrichtung aus einer Wärmespeicherplatte (1) besteht, die an der bei der Verwendung der Vorrichtung der Basisplatte (10) abgewandten Seite eine Aufnahmeöffnung (5) für ein Thermometer (2) besitzt, und wobei Mittel zur Fixierung des Thermometers (2) in der Aufnahmeöffnung (5) vorhanden sind, **dadurch gekennzeichnet, dass** an der Basisplatte (10) zugewandten Seite der Wärmespeicherplatte (1) eine Profilaussparung (4) in die Wärmespeicherplatte (1) eingearbeitet ist, wobei die Profilaussparung (4) im Wesentlichen der äußeren Kontur eines an der Basisplatte (10) vorhandenen Überstandes (14) zur Kopplung mit dem Beutel entspricht und deren Tiefe minimal der Höhe dieses Überstandes (14) entspricht, und dass die Wärmespeicherplatte (1) so ausgewählt wird, dass bei der Anwendung der Vorrichtung die Temperaturabsenkung an der Oberfläche der Wärmespeicherplatte (1) innerhalb von 2 min nicht mehr als 5°C beträgt.

2. Vorrichtung nach Anspruch 1, **dadurch gekennzeichnet, dass** die Wärmespeicherplatte (1) bei einer Temperaturabsenkung von 5°C eine Wärmemenge von mindestens 0,3 Wh abgeben kann.

3. Vorrichtung nach Anspruch 1 und 2, **dadurch gekennzeichnet, dass** die Wärmespeicherplatte (1) aus Metall besteht.

4. Vorrichtung nach Anspruch 3, **dadurch gekennzeichnet, dass** die Wärmespeicherplatte (1) aus Edelstahl oder Aluminium oder einer Aluminiumlegierung besteht.

5. Vorrichtung nach einem der Ansprüche 1 bis 4, **dadurch gekennzeichnet, dass** die Wärmespeicherplatte (1) ein Volumen von mehr als 50 cm³ besitzt.

6. Vorrichtung nach einem der Ansprüche 1 bis 5, **dadurch gekennzeichnet, dass** alle eingesetzten Teile aus bleifreien Materialien bestehen, wasserdicht sowie harz- und säurefest sind.

7. Vorrichtung nach einem der Ansprüche 1 bis 6, **dadurch gekennzeichnet, dass** die Grundfläche der Wärmespeicherplatte (1) der dem Körper zugewandten Fläche einer Spezialkleberschicht (13) der Basisplatte (10) entspricht.

8. Vorrichtung nach einem der Ansprüche 1 bis 7, **dadurch gekennzeichnet, dass** als Mittel zur Fixierung des Thermometers (2) in der Aufnahmeöffnung (5) im Umfang der Aufnahmeöffnung (5) eine Ringnut (6) für einen Sicherungsring (3) enthalten ist.

9. Vorrichtung nach einem der Ansprüche 1 bis 8, **dadurch gekennzeichnet, dass** in der Wärmespeicherplatte (1) eine Entwässerungsbohrung (7) enthalten ist, die in die Aufnahmeöffnung (5) mündet.

10. Vorrichtung nach einem der Ansprüche 1 bis 9, **dadurch gekennzeichnet, dass** als Mittel zur Fixierung des Thermometers (2) in der Aufnahmeöffnung (5) ein wärmeleitender Kleber eingesetzt wird.

11. Verfahren zur Anwendung einer Vorrichtung nach einem der Ansprüche 1 bis 10, **dadurch gekennzeichnet, dass** die Vorrichtung mit einer auf etwa 40°C erwärmten Wärmespeicherplatte (1) mindestens 2 min auf die Rückseite einer mit einer Spezialkleberschicht (13) belegten Basisplatte (10) einer Stomavorrichtung gelegt wird bis das enthaltene Thermometer (2) als Temperatur der Spezialkleberschicht (13) die Körpertemperatur anzeigt und die Vorrichtung nach Abkühlung der Wärmespeicherplatte (1) auf der Anzeige des Thermometers (2) um mindestens 5°C wieder entfernt wird.

## Claims

1. Device for fastening a base plate of stoma devices, having two-component coupling systems consisting of a detachable bag and of a separate self-adhesive base plate, to the skin of affected persons, wherein the device consists of a heat accumulator plate (1) which, on the side directed away from the base plate (10) during the use of the device, has a receiving opening (5) for a thermometer (2), and wherein means are present for fixing the thermometer (2) in the receiving opening (5), **characterized in that** a profiled cutout (4) is worked into the heat accumulator plate (1) on the side of the heat accumulator plate (1) directed towards the base plate (10), wherein the profiled cutout (4) corresponds substantially to the outer contour of a projection (14), present on the base plate (10), for coupling to the bag, and the depth of the profiled cutout (4) corresponds at least to the height of this projection (14), and **in that** the heat accumulator plate (1) is chosen such that, during the use of the device, the temperature decrease at the surface of the heat accumulator plate (1) is not more than 5°C within 2 min.

2. Device according to Claim 1, **characterized in that** the heat accumulator plate (1), at a temperature decrease of 5°C, can output a quantity of heat of at least 0.3 Wh.

3. Device according to Claims 1 and 2, **characterized in that** the heat accumulator plate (1) is made of metal.

4. Device according to Claim 3, **characterized in that** the heat accumulator plate (1) is made of stainless steel or aluminium or of an aluminium alloy.

5. Device according to one of Claims 1 to 4, **characterized in that** the heat accumulator plate (1) has a volume of more than 50 cm³.

6. Device according to one of Claims 1 to 5, **characterized in that** all of the parts used are made of lead-free materials and are watertight and resistant to resin and acid.

7. Device according to one of Claims 1 to 6, **characterized in that** the base area of the heat accumulator plate (1) corresponds to the surface, directed towards the body, of a special adhesive layer (13) of the base plate (10).

8. Device according to one of Claims 1 to 7, **characterized in that**, as means for fixing the thermometer (2) in the receiving opening (5), an annular groove (6) for a securing ring (3) is provided in the circumference of the receiving opening (5).

9. Device according to one of Claims 1 to 8, **characterized in that** a drainage bore (7), which leads into the receiving opening (5), is provided in the heat accumulator plate (1).

10. Device according to one of Claims 1 to 9, **characterized in that** a heat-conducting adhesive is used as means for fixing the thermometer (2) in the receiving opening (5).

11. Method for applying a device according to one of Claims 1 to 10, **characterized in that** the device is placed, with a heat accumulator plate (1) heated to approximately 40°C, for at least 2 min onto the rear face of a base plate (10) of a stoma device coated with a special adhesive layer (13), until the contained thermometer (2) indicates the temperature of the special adhesive layer (13) as body temperature, and the device is removed again after the heat accumulator plate (1) on the display of the thermometer (2) has cooled by at least 5°C.

## Revendications

1. Dispositif de fixation d'une plaque de base de dispositifs de stomie, ledit dispositif de fixation comportant des systèmes d'accouplement en deux parties, qui comprennent un sac amovible et une plaque de base autoadhésive séparée, sur la peau des personnes concernées, le dispositif comprenant une plaque d'accumulation de chaleur (1) qui possède une ouverture de réception (5) destinée à un thermomètre (2) sur le côté opposé à la plaque de base (10) lorsque le dispositif est utilisé, et des moyens étant prévus pour fixer le thermomètre (2) dans l'ouverture de réception (5), **caractérisé en ce qu'**un évidement profilé (4) est ménagé dans la plaque d'accumulation de chaleur (1) du côté de la plaque d'accumulation de chaleur (1) qui est dirigé vers la plaque de base (10), l'évidement profilé (4) correspondant sensiblement au contour extérieur d'un surplomb (14) présent sur la plaque de base (10) et destiné à l'accouplement au sac et la profondeur de l'évidement correspondant au moins à la hauteur de ce surplomb (14), et **en ce que** la plaque d'accumulation de chaleur (1) est choisie de telle sorte que, lorsque le dispositif est utilisé, la chute de température à la surface de la plaque d'accumulation de chaleur (1) ne dépasse pas 5 °C en 2 minutes.

2. Dispositif selon la revendication 1, **caractérisé en ce que** la plaque d'accumulation de chaleur (1) peut dégager une quantité de chaleur d'au moins 0,3 Wh lorsque la température baisse de 5 °C.

3. Dispositif selon les revendications 1 et 2, **caractérisé en ce que** la plaque d'accumulation de chaleur (1) est en métal.

4. Dispositif selon la revendication 3, **caractérisé en ce que** la plaque d'accumulation de chaleur (1) est en acier inoxydable ou en aluminium ou en un alliage d'aluminium.

5. Dispositif selon l'une des revendications 1 à 4, **caractérisé en ce que** la plaque d'accumulation de chaleur (1) a un volume de plus de 50 cm³.

6. Dispositif selon l'une des revendications 1 à 5, **caractérisé en ce que** toutes les pièces utilisées sont en matériaux sans plomb, sont étanches à l'eau et résistent aux résines et aux acides.

7. Dispositif selon l'une des revendications 1 à 6, **caractérisé en ce que** la surface de base de la plaque d'accumulation de chaleur (1) correspond à la surface, dirigée vers le corps, d'une couche adhésive spéciale (13) de la plaque de base (10).

8. Dispositif selon l'une des revendications 1 à 7, **caractérisé en ce qu'**une rainure annulaire (6) destinée à une bague de blocage (3) est ménagée en tant que moyen de fixation du thermomètre (2) dans l'ouverture de réception (5) sur le pourtour de l'ouverture de réception (5).

9. Dispositif selon l'une des revendications 1 à 8, **caractérisé en ce que** la plaque d'accumulation de chaleur (1) comporte un trou de drainage (7) qui débouche dans l'ouverture de réception (5).

10. Dispositif selon l'une des revendications 1 à 9, **caractérisé en ce qu'**une colle thermoconductrice est utilisée comme moyen de fixation du thermomètre (2) dans l'ouverture de réception (5).

11. Procédé d'utilisation d'un dispositif selon l'une des revendications 1 à 10, **caractérisé en ce que** le dispositif, pourvu d'une plaque d'accumulation de chaleur (1) chauffée à environ 40 °C, est placé pendant au moins 2 minutes au dos d'une plaque de base (10), pourvue d'une couche adhésive spéciale (13), d'un dispositif de stomie jusqu'à ce que le thermomètre (2) qu'il contient indique la température corporelle comme la température de la couche adhésive spéciale (13) et le dispositif est retiré après un refroidissement de la plaque d'accumulation de chaleur (1) d'au moins 5 °C sur l'affichage du thermomètre (2).
